# EUROPEAN PATENT APPLICATION

(11) **EP 3 900 709 A1**
(43) Date of publication of application: **27.10.2021**
(21) Application number: 19900875.6
(22) Date of filing: 20.12.2019
(51) Int. Cl.: A61K 9/51, A61K 8/11, A61K 8/73, A61Q 19/00, A23L 33/10

(54) **NANOCAPSULES COATED WITH CHITOSAN AND USE THEREOF**

(30) Priority: 20.12.2018 KR 20180166093; 18.12.2019 KR 20190169396
(71) Applicant: Skinmed Co., Ltd., Daejeon 34050 (KR)
(72) Inventor: CHOI, Won Il, Seoul 04500 (KR); KIM, Sung Hyun, Sejong-si 30098 (KR); SHIN, Yong Chul, Jinju-si, Gyeongsangnam-do 52817 (KR); LEE, Jeung Hoon, Daejeon 34124 (KR); KIM, Jin Hwa, Daejeon 34049 (KR); YUN, Young Sung, Jinju-si Gyeongsangnam-do 52731 (KR)
(74) Representative: Beckmann, Claus
(86) International application number: PCT/KR2019/018154
(87) International publication number: WO 2020/130699

(57) **Abstract**

The present invention relates to nanocapsules coated with chitosan, and a use thereof. A method for preparing nanocapsules having a particle size of 500 nm or less, or 300 nm, especially, 100 nm or less, and excellent stability has been established, and it has been ascertained that a poorly soluble drug is loaded in the nanocapsules prepared by the method, so that an excellent skin permeability rate of drug-containing nanocapsules, drug delivery into the skin achieved thereby, and efficacy caused by the drug are exhibited. In addition, an increase in the bioavailability of active ingredients in vivo through oral administration of the prepared nanocapsules is confirmed, and thus it is expected that nanocapsules coated with chitosan of the present invention are used to develop an excellent delivery system, of which the delivery efficiency of a poorly soluble drug or the active ingredients to animals such as humans, livestock, and companion animals is significantly increased, in the pharmaceutical field, cosmetics industry, food industry, and the like.

## Description

### Technical Field

The present disclosure relates to a nanocapsule coated with chitosan and a use thereof and, more specifically, to a nanocapsule in which a nanoparticle comprising a pluronic is coated with chitosan and a use thereof.

### Background Art

Nanoparticles are usually defined as particles of matter that are between 1 and 1,000 nm in diameter which is larger than an atom and smaller than a cell, and have emerged as substances that can create new application fields by taking advantage of the increase in surface area or permeable effect with the size decrease thereof. Particularly, they find increasing applications in the electronic parts industries utilizing electromagnetic properties, the medical and cosmetic industries utilizing drug absorption properties, the photocatalyst industry, the battery industry, etc.

A nanoparticle can be generally made of an inert material, such as gold, tin oxide, a polymeric material, e.g., albumin, etc. When applied to a biological system, such particles exhibit distinct physical, chemical, and biological features, such as increased membrane permeability, optical activation, control of aggregation at a molecular level, etc., compared to particles with larger diameters.

A nanocapsule is a ball-like, nanoscale shell having an empty inside in which various substances can be contained. A liposome, which is a kind of representative nanocapsule, is a spherical vesicle having at least one bilayer composed of amphipathic phospholipids and other components. Being capable of containing water-soluble drugs in the inner empty space thereof, liposomes are utilized in drug delivery. However, the application fields of such liposomes are limited because they are structurally unstable and have poor permeability. Thus, many trials have been made for improving stability and permeability in hollow capsules, resulting in a success in generating nanocapsules formed of polymers.

In the medicinal field, much research has been conducted into the use of nanocarriers containing various drugs therein in delivering drugs across cell membranes to exhibit medicinal effects of drugs. Reference is made to representative drug carriers. First, a liposome is a carrier formed of phospholipids and can contain both lipophilic and hydrophilic drugs. Liposomes are formed of biocompatible materials and free of toxicity and their surfaces can be suitably modified according to purposes. However, liposomes may be captured by the reticuloendothelial system in hepatocytes or splenocytes to undergo quick clearance from blood and destruction, so that a small number of the liposomes reach a target. In order to overcome the disadvantage, liposomes are subjected to surface modification with polyethylene glycol (PEG) to extend blood retention time in vivo or to treatment with various antibodies or ligands to increase targetability. A micelle serves as a carrier and is formed of copolymers having hydrophilic and hydrophobic chains. A micelle in water forms a spherical aggregate with the hydrophobic regions sequestered in the micelle center. Studies on increasing solubility and bioavailability of insoluble drugs by incorporating the insoluble drugs into the micelle core are ongoing (KWON, Ik Chan, 2010). In addition to the development of new materials of functional cosmetics for skin whitening, wrinkle reduction, antioxidation, and anti-aging, developing a technology for increasing the percutaneous absorption rate when applied to the skin is an important task in the cosmetics industry. The skin functions as a barrier to active ingredients, so that the active ingredients, even though having excellent efficacies, cannot exhibit their effects upon application to the skin. Thus, one of the biggest concerns in the cosmetics industry is to promote the absorption of active ingredients and maximize their effectiveness without irritating the skin. To this end, many methods have been suggested in order to promote the absorption of active ingredients. At present, active research for coverage from topical effects to systemic effects is ongoing, demonstrating that oil-in-water (O/W) formulations with a size of 500 nm or less are more likely to penetrate into the skin (KIM, Eun Ju et al., 2010).

A formulation approach has been predominantly studied over other methods for promoting percutaneous absorption rates. The formulation approach can be largely divided into five forms. A first approach is a pH-responsive polymeric hydrogel. That is, a cosmetic product prepared into a polymeric hydrogel form with pH responsiveness can benefit from the system that stably preserves an active ingredient unstable to external environments and allows the active ingredient to be quickly released and absorbed into the skin upon application to the skin. A second approach is a polymeric micelle which has hydrophilic polymers and hydrophobic polymers conjugated to each other in the form of block copolymers and thus is effective for dispersing a hydrophobic active ingredient in an aqueous liquid phase. Thirdly, a nano-emulsion, which is a kind of emulsion, ranges in particle size from 100 to 500 nm and does not undergo aggregation or mergence between particles in contrast to general emulsions, thus retaining long-term stability even in a low viscosity condition. Fourthly suggested is a liposome, which is composed of a lipid bilayer structurally similar to cell membranes, or intercellular lipids of the stratum corneum and thus fused to cell membranes to effectively deliver an active ingredient into cells. Finally, ethosomes and elastic liposomes are suggested. Being designed to increase skin permeability, compared to liposomes, the ethosomes and elastic liposomes have membranes which are more flexible and easier to deform (Chung, J.Y., et al., 2014).

In the food industry, nanoparticles or capsules are mainly used. They protect nutrient ingredients from external factors, such as light, oxygen, moisture, temperatures, etc., to decrease a loss of nutrient ingredients and have advantages including an increase in utility, physiological activity, stability, and target control, thus finding various applications even in high value products in the future. Being small in size and large in surface area, compared to conventional food substances, food substances to which nanotechnology has been applied exhibit improved permeability and retention time of the particles and capsules and thus are expected to increase in in-vivo absorption and bioavailability. In addition to improved solubility and dispersibility, the substances have the potential of passing through the difficult-to-cross cell lipid bilayer and thus can be applied to the effective use of functional materials (KIM, Sae Hoon et al., 2014).

Leading to the present disclosure, intensive and thorough research, conducted by the present inventors, into the development of a system for delivery of physiologically effective substances, resulted in the establishment of a method for manufacturing nanocapsules that are of excellent stability and have a particle size of 500 nm or less, and especially 200 nm or less, and in the finding that when manufactured by the method, the nanocapsules having insoluble drugs loaded therein exhibited high skin permeability and guaranteed transdermal drug delivery and drug efficacy in the body. It was also found that when the manufactured nanocapsules were administered orally, the active ingredients increased in bioavailability.

As a related art, Korea Patent Number 1698809 discloses a multilayer nanoparticle comprising an insoluble drug, a pluronic, and chitosan, with the additional inclusion of a glycol-based compound in the drug-containing first core and a polyoxyether compound or polyoxy castor oil compound in the poloxamer-containing second layer, which is configurationally different from the nanoparticle composed only of an insoluble drug and a pluronic according to the present disclosure. In addition, Korean Patent Number 1748127 discloses a chitosan-coated nanoparticle comprising a drug and a pluronic (poloxamer), which is prepared by adding a pluronic and PLGA to a drug to give a nanoparticle and coating the nanoparticle with chitosan. The nanoparticle is configurationally different from the nanocapsule obtained by coating a nanoparticle composed only of a drug and a pluronic with chitosan. Nowhere is the excellent skin penetrating effect of the nanocapsule of the present disclosure described in the document.

The non-patent document Escobar-Chavesz, J.J., et al., (2006) discloses pharmaceutical formulations, nanoparticles, temperature responsiveness, and dermal delivery with respect to a pluronic gel, stating the skin permeability of chitosan. However, the nanocapsule in which a nanoparticle including a drug and a pluronic is coated with chitosan, and the increased skin permeability effects thereof are not disclosed anywhere in the document.

### Disclosure of Invention

### Technical Problem

An aspect of the present disclosure is to provide a chitosan-coated nanocapsule and a method for manufacturing same.

Another aspect of the present disclosure is to provide a composition comprising the nanocapsule for use in various purposes.

### Solution to Problem

The present disclosure pertains to a nanocapsule, in which a nanoparticle comprising an active agent and a pluronic is coated with chitosan.

The nanocapsule may be manufactured through a first step of preparing a reaction solution by dissolving an active agent and a pluronic in an organic solvent and reacting the same at room temperature; a second step of preparing a nanoparticle by dropwise adding the reaction solution of the first step to distilled water, continuously stirring the same, and removing the organic solvent of the reaction solution through natural evaporation; and a third step of coating the nanoparticle of the second step by adding chitosan thereto.

The active agent may be used in an amount of 0 (exclusive) to 20 parts by weight (inclusive), based on 100 parts by weight of the pluronic.

The active agent may be at least one selected from the group consisting of an anticancer agent, an immunosuppressant, an antioxidant, an anti-inflammatory agent, an anti-wrinkling agent, an anti-hair loss preparation, a wound healing agent, a skin whitening agent, a nutritional supplement, an immunogen, a protein as a therapeutic agent, a revascularization agent, an antifungal agent, an antibiotic, an antiviral agent, a sedative, an analgesic, an anti-aging agent, an anti-wrinkling agent, a skin whitening agent, a skin depigmenting agent, an ultraviolet blocking agent, a dye, a colorant, a deodorizing agent, and an air freshener.

The active agent may be a fat-soluble or insoluble drug. The fat-soluble or insoluble drug may be at least one selected from the group consisting of fat-soluble or insoluble anticancer agents including paclitaxel, docetaxel, and tetradrine, fat-soluble or insoluble immunosuppressants including cyclosporin A and dexamethasone, fat-soluble or insoluble antioxidants including tocopheryl acetate, astaxanthin, curcumin, and ascorbyl palmitate, fat-soluble or insoluble anti-inflammatory agents including dexpanthenol and caffeic acid phenethyl ester (CAPE), fat-soluble or insoluble anti-wrinkle agents including retinyl palmitate, fat-soluble or insoluble anti-hair loss preparations including minoxidil and finasteride, fat-soluble or insoluble wound healing agents including a Centella asiatica extract and beta-sitosterol, fat-soluble or insoluble skin whitening agents including ascorbyl tetraisopalmitate, and fat-soluble or insoluble nutritional supplements including tripeptide collagen.

The active agent may be a water-soluble drug which may be at least one selected from the group consisting of: water-soluble anticancer agents including doxorubicin; water-soluble anti-inflammatory agents including phospholipase A2 (PLA2); water-soluble immunogens including ovalbumin; water-soluble proteins as therapeutic agents including bovine serum albumin; water-soluble wound healing agents including fibroblast growth factor (b-FGF); and water-soluble revascularization agents including vascular endothelial growth factor (VEGF).

The pluronic may be at least one selected from the group consisting of pluronic L35, pluronic L43, pluronic L44, pluronic L64, pluronic F68, pluronic P84, pluronic P85, pluronic F87, pluronic F88, pluronic F98, pluronic P103, pluronic P104, pluronic P105, pluronic F108, pluronic P123, and pluronic F127.

The nanoparticle may range in size from 5 to 80 nm and preferably from 5 to 50 nm as measured at 32.5-37°C.

The chitosan may have a molecular weight of 3-100 kDa.

The chitosan may be contained in an amount of 0.001-200 parts by weight, based on 100 parts by weight of the pluronic.

The nanocapsule may have a particle size of 700 nm or less as measured at 32.5-37°C. The particle size of the nanocapsule may be preferably 30-500 nm, more preferably 30-300 nm, and most preferably 30-100 nm at 32.5-37°C.

The organic solvent in the first step may be at least one selected from the group consisting of acetone, DMSO (dimethyl sulfoxide), ethanol, acetonitrile, tetrahydrofuran, chloroform, and dichloromethane.

In the second step, the distilled water may be used in a volume 4-fold larger than that of the organic solvent of the first step.

The nanocapsule may increase in skin permeability by 2 fold, preferably by 5 fold, more preferably by 10 fold, and further more preferably by 14 fold, compared to the active agent alone.

In addition, the present disclosure pertains to a drug delivery system, a cosmetic composition, a health functional food composition, a composition for medical instruments, and a composition for daily necessities, each of which comprises the nanocapsule.

Below, a detailed description will be given of the present disclosure.

The present disclosure pertains to a chitosan-coated nanocapsule and specifically to a nanocapsule in which a nanoparticle comprising an active agent and a pluronic is coated with chitosan.

As used herein, the term "nanocapsule" refers to a hollow spherical capsule in a nano scale, which can load various substances, e.g., an active agent, in the empty core thereof.

The nanocapsule of the present disclosure can be prepared using a method known in the art, preferably a nanoprecipitation and membrane resuspension method, and more preferably a nanoprecipitation method.

Most preferably, the nanocapsule may be manufactured using a process comprising: a first step of preparing a reaction solution by dissolving an active agent and a pluronic in an organic solvent and reacting the same at room temperature to give a reaction solution; a second step of preparing a nanoparticle by dropwise adding the reaction solution of the first step to distilled water, continuously stirring the same, and removing the organic solvent of the reaction solution through natural evaporation; and a third step of coating the nanoparticle of the second step by adding chitosan thereto.

The organic solvent in the first step may be at least one selected from the group consisting of acetone, DMSO (dimethyl sulfoxide), ethanol, acetonitrile, tetrahydrofuran, chloroform, and dichloromethane, but without limitations thereto. The organic solvent is preferably at least one selected from the group consisting of acetone, tetrahydrofuran, ethanol, and acetonitrile and more preferably acetone.

In the second step, distilled water may be used in a volume 2-10 times, preferably 2-5 times, and more preferably 4 times as much as that of the organic solvent of the first step. If the distilled water is used at less than twice the volume of organic solvents, precipitation may occur in part, and if it is more than 10 times, the chitosan coating may be unstable or the concentration of nanocapsules may be diluted, undesirably requiring an additional enrichment process.

The active agent is an effective substance with water solubility or fat solubility and may be at least one selected from the group consisting of an anticancer agent, an immunosuppressant, an antioxidant, an anti-inflammatory agent, an anti-wrinkle agent, an anti-hair loss preparation, a wound healing agent, a skin whitening agent, a nutritional supplement, an immunogen, a protein as a therapeutic agent, a revascularization agent, an antifungal agent, an antibiotic, an anti-viral agent, a sedative, an analgesic, an anti-aging agent, an anti-wrinkle agent, a skin whitening agent, a skin depigmenting agent, an ultraviolet blocking agent, a dye, a colorant, a deodorizing agent, and an air freshener, but without limitations thereto.

The active agent may be contained in a minimum amount necessary to ensure the efficacy and effectiveness thereof to 20 parts by weight, that is, in an amount of 0 parts by weight (exclusive) to 20 parts by weight, based on 100 parts by weight of the pluronic. Preferably, the active agent may be contained in an amount of 0 parts by weight (exclusive) - 10 parts by weight. When the amount of the active agent exceeds 20 parts by weight, the nanoparticles become too large in size or cannot carry the entire active agent, failing to transfer an exactly effective amount of the active agent.

In the present disclosure the "pluronic" (poloxamer) is a hydrophilic polymer characterized by temperature responsiveness, and derivatives with various HLB (hydrophile-lipophile balance) values exist. The pluronic may be at least one selected from pluronics with an HLB of 8-29, for example, from a group consisting of pluronic L35, pluronic L43, pluronic L44, pluronic L64, pluronic F68, pluronic P84, pluronic P85, pluronic F87, pluronic F88, pluronic F98, pluronic P103, pluronic P104, pluronic P105, pluronic F108, pluronic P123, and pluronic F127, and preferably from pluronics with an HLB of 15-29, for example, from a group consisting of pluronic L35, pluronic L44, pluronic L64, pluronic F68, pluronic P85, pluronic F87, pluronic F88, pluronic F98, pluronic P105, pluronic F108, and pluronic F127, but without limitations thereto.

The nanoparticle comprises an active agent and a pluronic and is temperature sensitive, thus varying in size depending on the temperature at which measurement is made. In detail, the particle size may increase with the decrease of temperature.

The nanoparticle may be 5-80 nm and preferably 5-50 nm in size as measured at 32.5-37°C.

As used herein, the term "chitosan" refers to a polysaccharide that is produced by partial deacetylation of chitin. Chitosan is a non-toxic, biocompatible, and highly biodegradable polymer with high hydrophilicity and mucoadhesiveness. Tending to become highly soluble and be positively charged in an acidic environment, chitosan is easily apt to attach to sites like mucous membranes. In addition, chitosan is of antimicrobial activity and has a hemostatic effect. Generally, chitosan is highly soluble in an acidic solution such as acetic acid, lactic acid, etc. When applied to the human body, a solution of chitosan in an acid may cause skin irritation or a disorder due to a pH change in the human body.

In contrast, the chitosan of the present disclosure, which is easily dissolved in water, can overcome the problems encountered with the use of the chitosan that is dissolved in an acidic solution. The chitosan may have a molecular weight of 3-100 kDa, preferably 3-20 kDa, and more preferably 3-10 kDa. Chitosan having a molecular weight exceeding 100 kDa is undesired due to its poor solubility in water.

The chitosan may be contained in an amount of 200 parts by weight or less, preferably in an amount of 0.001-200 parts by weight, and more preferably in an amount of 0.001-100 parts by weight, based on 100 parts by weight of the pluronic. Less than 0.001 parts by weight of the chitosan is too small to sufficiently coat the nanoparticle, so that it is difficult to exhibit positive charges on the surface of the nanoparticle. When the chitosan is used in an amount more than 200 parts by weight, the nanocapsule becomes too large or undergoes partial precipitation.

The nanocapsule exhibits temperature sensitivity and increases in particle size with the decrease of temperature. The nanocapsule has a particle size of 1,000 nm or less as measured at 10°C and decreases in particle size at temperatures higher than 10°C.

The nanocapsule is preferably 700 nm or less in particle size at 32.5-37°C, more preferably 30-500 nm in particle size, further more preferably 30-300 nm in particle size, and most preferably 30-100 nm in particle size. Given a particle size exceeding 700 nm, the nanocapsule is poor in skin permeability when applied to the skin.

The nanocapsule can load various active agents to the empty core thereof. In addition, the nanocapsule swells at low temperatures due to the temperature sensitivity thereof to allows the insertion of active agents between the pluronic materials responsible for the constitution of the nanocapsule, whereby fat-soluble and water-soluble active agents can both be retained.

Being coated with chitosan, the nanocapsules are positively charged on the surface thereof and thus may increase in skin permeability and mucoadhesiveness.

The skin permeability of the chitosan-coated nanocapsule is remarkably higher than those of polymer capsules (PluNC) lacking chitosan and six or more times higher than those of most commercially available liposome formulations and may increase by two fold or more, preferably by five fold or more, more preferably by 10 fold or more, and further more preferably by 14 fold or more, compared to nanocapsules treated with an active agent alone.

The nanocapsule is made by physical bonding between the pluronics constituting the nanoparticle and the chitosan coated on the nanoparticle and does not require a separate polymer manufacturing process, unlike nanoparticles manufactured using the pluronic-chitosan polymers prepared through chemical bonds between pluronics and chitosan, and thus the toxicity of the binder used for preparing the polymer needs not be taken into consideration.

Moreover, the present disclosure pertains to a drug delivery system comprising the nanocapsule.

In the present disclosure, the "drug delivery system" refers to a system for delivering a therapeutically effective drug to an in-vivo site in need thereof. Designed to effectively transfer a requisite amount of a drug to a tissue in need thereof, the drug delivery system may be understood as a drug composition, a drug modality, a formulation method, or a drug formulation.

The drug delivery system may be a nanocapsule containing a therapeutically efficacious drug therein.

The drug may be at least one selected from the group consisting of an anticancer agent, an immunosuppressant, an antioxidant, an anti-inflammatory agent, an anti-wrinkle agent, an anti-hair loss preparation, a wound healing agent, a skin whitening agent, a nutritional supplement, an immunogen, a protein as a therapeutic agent, a revascularization agent, an antifungal agent, an antibiotic, an anti-viral agent, a sedative, an analgesic, an anti-aging agent, an anti-wrinkle agent, a skin whitening agent, a skin depigmenting agent, an ultraviolet blocking agent, a dye, a colorant, a deodorizing agent, and an air freshener, but without limitations thereto.

Examples of the anti-fungal agent include polyenes such as amphotericin B, nystatin, fungicidin, etc., azoles such as ketoconazole, itraconazole, etc., allylamines such as butenafine, terbinafine, naftifine, etc., echinocandins such as anidulafungin, caspofungin, etc., and other anti-fungal agents such as aurones, benzoic acid, ciclopirox, flucytosine, griseofulvine, etc., but are not limited thereto.

The antibiotic may be selected from penicillins, cephalosporins, polymyxins, sulfonamides, quinolines, rifampicin, aminoglycosides, macrolides, tetracyclines, but are not limited thereto.

The anti-viral agent may be: an anti-influenza virus agent, e.g., amantadine, rimantadine, oseltamivir, zanamivir, etc.; an anti-herpes virus agent, e.g., vidarabine, acyclovir, foscarnet, etc.; an anti-hepatitis B virus agent, e.g., lamivudine, entecavir, tenofovir, etc.; or an anti-HIV agent, e.g., zidovudine, didanosine, zalcitabine, efavirenz, rilpivirine, saquinavir, ritonavir, raltegravir, elvitegravir, dolutegravir, enfuvirtide, etc., but is not limited thereto.

The sedative may be zolpidem, diazepam, or morphine, but is not limited thereto.

Within the scope of the analgesic, acetaminophen drugs, nonsteroidal anti-inflammatory drugs, morphine, fentanyl, oxycodone, and hydromorphone fall, but with no limitations thereto.

Examples of the wound healing agent include, but are not limited to, centella asiatica, collagen, and epithelial growth factor (EGF).

The anti-inflammatory agent may be meloxicam, silibinin, indomethacin, propolis, caffeic acid phenethyl ester, etc., but without limitations thereto.

The anticancer agent may be exemplified by paclitaxel, estrogen, doxorubicin, 5-fluoro uracil, popinavir, nimusulide, progesterone, repaglinide, tetracycline, all-trans retinoic acid, luteoline, a vascular endothelial growth factor receptor (VEGFR) inhibitor, a Wnt/β-catenin modulator, a hedgehog inhibitor, and a PI3K/Akt/mTOR modulator, but without limitations thereto.

The immunosuppressant may be cyclosporin A, tacrolimus, methotrexate, rapamycin, or sirolimus, but without limitations thereto.

The anti-hair loss preparation, which refers to a substance effective for preventing hair loss or promoting hair regrowth, may include finasteride, minoxidil, cyclosporin A, a natural anti-hair loss preparation, e.g., a *Coicis semen* extract, a *Rubus coreanus* extract, a *Glycyrrhiza radix* extract, a *Thuja orientalis* extract, an *Angelical radix* extract, a *Cornus officinalis* extract, etc., or a peptide which is effective for promoting hair regrowth, but is not limited thereto.

The active agent may be a fat-soluble or insoluble drug which may be at least one selected from the group consisting of fat-soluble or insoluble anticancer agents including paclitaxel, docetaxel, and tetradrine, fat-soluble or insoluble immunosuppressants including cyclosporin A and dexamethasone, fat-soluble or insoluble antioxidants including tocopheryl acetate, astaxanthin, curcumin, and ascorbyl palmitate, fat-soluble or insoluble anti-inflammatory agents including dexpanthenol and caffeic acid phenethyl ester (CAPE), fat-soluble or insoluble anti-wrinkle agents including retinyl palmitate, fat-soluble or insoluble anti-hair loss preparations including minoxidil and finasteride, fat-soluble or insoluble wound healing agents including a Centella asiatica extract and beta-sitosterol, fat-soluble or insoluble skin whitening agents including ascorbyl tetraisopalmitate, and fat-soluble or insoluble nutritional supplements including tripeptide collagen.

The active agent may be a water-soluble drug which may be at least one selected from the group consisting of: water-soluble anticancer agents including doxorubicin; water-soluble anti-inflammatory agents including phospholipase A2 (PLA2); water-soluble immunogens including ovalbumin; water-soluble proteins as therapeutic agents including bovine serum albumin; water-soluble wound healing agents including fibroblast growth factor (b-FGF); and water-soluble revascularization agents including vascular endothelial growth factor (VEGF).

The drug delivery system may comprise the nanocapsule and a pharmaceutically acceptable excipient.

According to the conventional methods, the drug delivery system may be formulated into: oral formulations such as powders, granules, tablets, capsules, suspensions, emulsions, syrups, aerosols, etc.; agents for external use; suppositories; and sterile injection solutions. Examples of the carrier, excipient, and diluent to be contained in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. For formulation, commonly used fillers, extenders, binders, humectants, disintegrants, diluents such as surfactants or excipients may be used. Examples of solid formulations for oral administration may include tablets, pills, powders, granules, capsules, etc. These solid formulations are prepared, for example, by adding at least one excipient, e.g., starch, calcium carbonate, sucrose, lactose, gelatin, etc., to the nanocapsule. Additionally, lubricants such as magnesium stearate, talc, etc., may be used in addition to the simple excipients. Examples of liquid preparations for oral administration include suspensions, oral solutions, emulsions, syrups, etc., and various kinds of excipients, e.g., humectants, sweeteners, fragrances, preservatives, etc., may be used in addition to simple diluents such as water and liquid paraffin. Preparations for parenteral administration include sterile aqueous solutions, non-aqueous solvents, suspensions, emulsions, lyophilized preparations, and suppositories. Examples of non-aqueous solvents for suspensions may include propylene glycol, polyethylene glycol, a vegetable oil such as olive oil, an injectable ester such as ethylolate, etc. Examples of suppository bases include Witepsol, Macrogol, Tween 61, cacao butter, laurinum, glycerogelatin, etc.

In addition, the external skin preparations may be prepared in the form of ointments, lotions, sprays, patches, creams, gelling agents, or gels, but are not particularly limited thereto. The external skin preparation may contain a penetration enhancer, for example, particularly, dimethyl sulfoxide, dimethylacetamide, dimethylformamide, a surfactant, an alcohol, an acetone, a propylene glycol, or polyethylene glycol, in a non-limiting sense. The frequency of application of the external skin preparation may significantly vary depending on various factors including the age, sex, and weight of a subject to be treated, a specific disease or pathological condition to be treated, severity of the disease or pathological condition, administration route, and judgement of a prescriber. The external skin preparation is suggested to be applied ten times per day to per month, preferably four times per day to per week, more preferably three times per day to per week, even more preferably once or twice per day.

The drug delivery system of the present disclosure may be administered to mammals such as mice, domestic animals, humans, companion animals, etc. via various routes. All modes of administration can be envisaged. For example, administration may be made through oral, rectal, intravenous, intramuscular, subcutaneous, dermal, dural, or intracerebroventricular routes, with preference for transdermal administration.

The present disclosure pertains to a cosmetic composition comprising the nanocapsule.

The nanocapsule may contain a functional cosmetic substance that has a function of skin whitening, wrinkle reduction, anti-oxidation, anti-aging, anti-inflammation, UV blocking, etc.

Examples of the skin whitening substance include a paper mulberry extract, arbutin, ethyl ascorbyl ether, an oil-soluble licorice extract, ascorbyl glucoside, niacinamide, α-bisabolol, and ascorbyl tetraisopalmitate, but are not limited thereto.

Examples of the wrinkle-related functional substance include, but are not limited to, vitamin A, vitamin A derivatives (retinyl palmitate, retinyl acetate, and so on), adenosine, and polyethoxylated retinamide.

The antioxidation functional substance may be exemplified by vitamin A, vitamin A derivatives, vitamin E, vitamin E derivatives, carotene, lycopene, lutein, coenzyme Q10, and astaxanthin, but are not limited thereto.

The cosmetic composition may include an auxiliary agent commonly used in the cosmetic field, such as a hydrophilic or lipophilic gelling agent, a hydrophilic or lipophilic active agent, a preservative, an antioxidant, a solvent, a fragrance, a filler, a blocker, a pigment, a deodorant, a dye, and the like in addition to the nanocapsules.

The amount of the auxiliary agent is at a level acceptable in the art. In any case, the auxiliary agents and their ratios might be selected so as not to adversely affect desired properties of the cosmetic composition according to the present disclosure.

The cosmetic composition may be prepared into at least one formulation selected from the group consisting of a lotion, a skin softener, a skin toner, an ampoule, an astringent, a cream, a foundation, an essence, a pack, a mask pack, a soap, a body cleanser, a cleansing foam, a shampoo, a rinse, a hair treatment, a hair oil, a body oil, and a body lotion., but without limitations thereto.

The cosmetic composition may be used every day, and may also be used for an indefinite period. Preferably, the amount, frequency and duration of use may be adjusted according to the user's age, skin condition, or skin type.

In addition, the present disclosure relates to a health functional food composition comprising the nanocapsule.

The nanocapsule may include a health functional food material.

The health functional food substance may be a vitamin, a mineral, a probiotic, a bioactive peptide, an antioxidant, a vegetable sterol, a plant extract, coenzyme Q10, omega-3, astaxanthin, and the like. Preferably, it may be collagen tripeptide, red ginseng oil, astaxanthin, and omega-3, with no limitations thereto.

The health functional food composition may include the nanocapsule and a sitologically acceptable supplemental additive.

The health functional food composition of the present disclosure may be in the form of tablets, capsules, pills, or liquids, and examples to which the nanocapsules of the present disclosure can be added include various foods, beverages, gums, teas, Vitamin complexes, health functional foods, etc.

Another aspect of the present disclosure provides a medical device comprising the nanocapsule of the present disclosure.

The medical device may be a filler, a wound coating agent, a bone graft material, an implant coating agent, an embolization aid, a diagnostic agent, or the like.

Another aspect of the present disclosure provides a composition for daily necessities, comprising the nanocapsule of the present disclosure.

The composition for daily necessities may be a dye, a colorant, a deodorizing agent, and an air freshener, but is not limited thereto.

### Advantageous Effects of Invention

The present disclosure relates to a chitosan-coated nanocapsule and a use thereof. In the present disclosure, a method for manufacturing a nanocapsule having a particle size of 500 nm or less, and particularly 200 nm or less, and excellent stability has been established, and when manufactured by the method, the nanocapsules having insoluble drugs loaded therein exhibited high skin permeability and guaranteed transdermal drug delivery and drug efficacy in the body. It was also found that when the manufactured nanocapsules were administered orally, the active ingredients increased in bioavailability.

Hence, the chitosan-coated nanocapsules of the present disclosure are expected to find applications in developing a delivery system that can deliver insoluble drugs or effective ingredients at remarkably increased efficiency to the human body and animals such as livestock, companion animals, etc., in the medical, cosmetic, and food industries.

### Brief Description of Drawings

FIG. 1 shows diameters of nanoparticles prepared according to pluronic types and temperatures.
FIG. 2 shows characteristics of chitosan-coated nanocapsules according to pluronic types, as analyzed for (a) morphological property, (b) size, (c) polydispersity, and (d) surface charge.
FIG. 3 shows characteristics of chitosan-coated nanocapsules according to molecular weights of chitosan, as analyzed for (a) size, (b) polydispersity, (c) surface charge, and (d) morphology.
FIG. 4 shows characteristics of chitosan-coated nanocapsules according to solvent types, as analyzed for (a) size, (b) polydispersity, and (c) surface charge.
FIG. 5 shows particle sizes according to preparation processes for the chitosan-coated nanocapsules having drugs loaded therein of the present disclosure.
FIG. 6 shows particle sizes of the chitosan-coated nanocapsules having drugs loaded therein according to mixing ratios of a solvent and deionized water when they were manufactured using nanoprecipitation.
FIG. 7 shows cytotoxicity of chitosan-coated nanocapsules according to the present disclosure.
FIG. 8 shows characteristics of chitosan-coated nanocapsules having paclitaxel and docetaxel loaded therein according to load amounts of the drugs, as analyzed for (a) size, (b) polydispersity, and (c) surface charge.
FIG. 9 shows characteristics of chitosan-coated nanocapsules having (a) cyclosporin A and (b) dexamethasone loaded therein according to load amounts of the drugs, as analyzed for size, polydispersity, and surface charge.
FIG. 10 shows characteristics of chitosan-coated nanocapsules having (a) retinyl palmitate and (b) tocopheryl acetate loaded therein according to load amounts of the drugs, as analyzed for size, polydispersity, and surface charge.
FIG. 11 shows characteristics of chitosan-coated nanocapsules having (a) minoxidil and (b) finasteride loaded therein according to load amounts of the drugs, as analyzed for size, polydispersity, and surface charge.
FIG. 12 shows size and dispersity characteristics of nanocapsules according to temperatures.
FIG. 13 shows characteristics of chitosan-coated nanocapsules having doxorubicin loaded therein according to loading amounts of the drug.
FIG. 14 shows characteristics of chitosan-coated nanocapsules having ovalbumin loaded therein according to loading amounts of the drug.
FIG. 15 shows characteristics of chitosan-coated nanocapsules having bovine serum albumin loaded therein according to loading amounts of the drug.
FIG. 16 shows whether chitosan-coated nanocapsules containing cyclosporin A (CsA@ChiNC), Nile red (Nile red@ChiNC), or pyrene (pyrene@ChiNC) precipitate or not.
FIG. 17 shows characteristics of chitosan-coated nanocapsules having drugs (CsA and RP) loaded therein before and after freeze drying.
FIG. 18 shows skin permeability of drugs according to chitosan types.
FIG. 19 shows skin permeability of chitosan-coated nanocapsules having a drug (Nile red) loaded therein according to the present disclosure.
FIG. 20 shows effects of chitosan-coated nanocapsules having a drug (Nile red) loaded therein of the present disclosure on the delivery of the drug into the skin.
FIG. 21 shows hair regrowth effects according to the transdermal delivery of a drug (cyclosporin A, CsA) by the chitosan-coated nanocapsule having the drug loaded therein of the present disclosure.

### Best Mode for Carrying out the Invention

Hereinafter, preferred embodiments of the present disclosure will be explained in detail. However, the present disclosure is not limited to the embodiments described herein and may be embodied in other forms. Rather, the embodiments are provided to make the disclosure introduced herein thorough and complete and to sufficiently elucidate the spirit of the present disclosure to those skilled in the art.

### <EXAMPLE 1: Preparation of Chitosan-Coated Nanocapsule>

### Example 1-1. Optimization for preparation of nanoparticle according to kind of pluronic

A pluronic (poloxamer) is a nonionic triblock copolymer composed of a central hydrophobic chain of poly(propylene oxide) (PPO) flanked by two hydrophilic chains of poly(ethylene oxide) (PEO), that is, PEO-PPO-PEO, which is a representative temperature-responsive polymer characterized by a temperature-dependent reversible transition of internal structures. Pluronic derivatives with various HLB (hydrophile-lipophile balance) values exist depending on mole numbers of PEO and PPO blocks therein. The HLB in pluronics may have influence on the preparation of nanoparticles from the pluronics. Thus, sizes of the nanoparticles were measured according to kinds of pluronics.

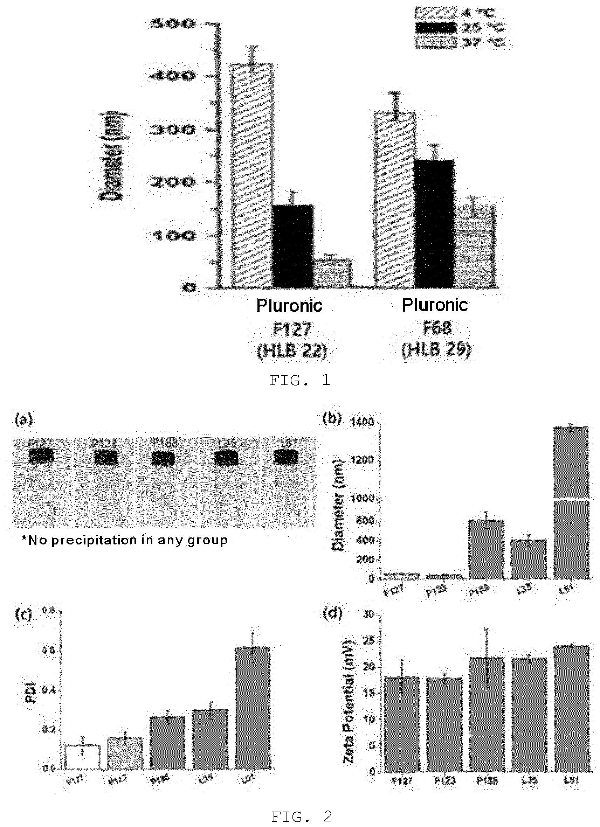

Nanoparticles were prepared using pluronics with an HLB of 2-29. In detail, 20 mg of a pluronic was dissolved in 1 ml of acetone to give a reaction solution. Drops of the reaction solution were slowly added to 4 ml of deionized water which were being stirred at 530 rpm. After reaction at room temperature for 12 hours or longer, the acetone was removed by natural vaporization to afford nanoparticles composed of a pluronic. The nanoparticles thus obtained were analyzed for sizes, using a particle size analyzer (Zetasizer, Nono-Zs, Malvern) and transmission electron microscopy. The result is depicted in FIG. 1.

As can be seen in FIG. 1, nanoparticles composed of pluronic F127 were measured to have a diameter of 200 nm or less at 25°C and to range in diameter from 5 nm to 80 nm at 32.5°C-37°C, with an average diameter of 50 nm. As for pluronic F68, its diameter was measured to be 300 nm or less at 25°C and 200 nm or less at 32.5°C-37°C.

In addition to pluronic F127 and pluronic F68, nanoparticles composed of pluronics with an HLB of 2-29 were found to have a diameter of 200 nm at 32.5°C-37°C.

As described above, it was understood that particle sizes can be controlled according to temperatures using pluronics. Nanocapsules with a diameter of 100 nm or less were constructed. To this end, the diameter of nanoparticles was optimized to 5-80 nm and preferably 5-50 nm, using pluronics with HLB 2-29.

### EXAMPLE 1-2. Optimization for preparation of nanocapsule according to kind of pluronic

Optimal conditions for preparation of nanocapsules were examined according to kinds of pluronics. In this regard, nanoparticles were prepared using pluronics corresponding to HLB 2-29 in Table 1, below and coated with chitosan to afford nanocapsules.

Briefly, 20 mg of each pluronic was dissolved in 1 ml of acetone and stirred at room temperature for 2 hours. Then, the polymer solution was slowly dropped into 4 ml of deionized water which was being stirred at 400 rpm. From the polymer nanoparticles thus obtained, acetone was removed for 6 hours under a hood. Finally, 20 mg of chitosan with a degree of deacetylation of 90% and a molecular weight of 10 kDa was added to each polymer nanoparticle, followed by stirring at room temperature for 2 hours to afford chitosan-coated nanocapsules. The chitosan-coated nanocapsules (ChiNC) constructed according to kinds of pluronics, were analyzed for size, dispersibility, and surface charge, using an electrophoretic light scattering spectrophotometer (ELS-Z2, Otsuka), and the results are depicted in FIG. 2.

**TABLE 1**

| Pluronic | Mw (Da) | HLB value |
|---|---|---|
| F68(P188) | 8,400 | 29 |
| F127(P407) | 12,600 | 22 |
| L35 | 1, 900 | 19 |
| P123 | 5,750 | 8 |
| L81 | 2,750 | 2 |

As can be seen FIG. 2, chitosan-coated nanocapsules (ChiNC) constructed from five pluronics different in HLB (Hydrophilic and Lipophilic Balance) indices (F127, P123, P188, L35, and L81) by nanoprecipitation were observed to be stable, with no precipitates generated, for all kinds of pluronics.

When prepared from F127 and P123, chitosan-coated nanocapsules were measured to stably have a diameter of 100 nm or less, for example, a diameter ranging from 30 nm to 80 nm, with an average diameter of 60 nm, at 32.5°C-37°C. The chitosan-coated nanocapsules prepared from P188 and L35 ranged in diameter from 209 nm to 688 nm, with an average diameter of 500 nm. The chitosan-coated nanocapsules prepared from L81 were up to 1.4 µm in diameter. Chitosan-coated nanocapsules prepared from F127, P123, P188, and L35 had a degree of dispersion of 0.3 or less, generally exhibiting monodispersity. The chitosan-coated nanocapsules prepared from L81 were in micro sizes, showing somewhat large dispersity. For surface charge, about +20 mV was measured on average because the pluronic capsules were stably coated with chitosan.

From the results, it was understood that pluronics ranging in HLB from 8 to 29 were all suitable for optimizing chitosan-coated nanocapsule formulations. Depending on pluronic kinds and temperatures, chitosan-coated nanocapsules can be formed to have a diameter of 700 nm or less, 30-500 nm, 30-300 nm, or 30-100 nm.

Although not in nano sizes, microcapsules could be constructed even at an HLB index of 8 or less, demonstrating that chitosan-coated capsules in nano and micro sizes can be constructed as needed for, for example, application to topical loci.

### Example 1-3. Optimization for preparation of chitosan-coated nanocapsule according to molecular weight of chitosan

On the basis of the results of Examples 1-1 and 1-2, optimal conditions were established in consideration of pluronic conditions for preparation of chitosan-coated nanocapsules according to chitosan molecular weights (3 kDa-100 kDa).

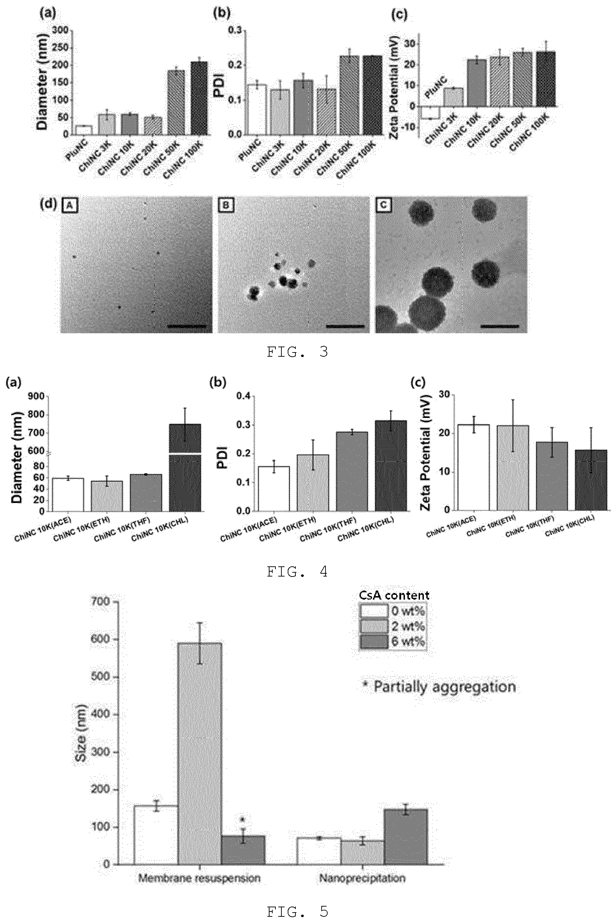

For preparation of the chitosan-coated nanocapsules of the present disclosure, the biocompatible material pluronic F127 (poloxamer 407), which had been approved by the FDA, was employed. Chitosan-coated nanocapsules were prepared using nanoprecipitation. In brief, 20 mg of pluronic F127 was dissolved in 1 ml of acetone to give a reaction solution which was then slowly dropped into 4 ml of deionized water that was being stirred at 530 rpm. During reaction at room temperature for 12 hours, the acetone was removed through natural vaporization to obtain nanoparticles composed of the pluronic (PluNC). Chitosan was added to the PluNC and mixed at room temperature for one hour or longer to afford chitosan-coated nanocapsules (ChiNC) of the present disclosure. In this regard, the PluNC was coated with chitosan having molecular weights of 3, 10, 20, 50, and 100 kDa. The ChiNC prepared according to molecular weights of chitosan was analyzed for morphology, size, polydispersity index (PDI), and surface charge by a particle size analyzer (Zetasizer, Nono-Zs, Malvern) and transmission electron microscopy. The results are depicted in FIG. 3.

Chitosan was mixed in an amount of 0.001-200 parts by weight with the pluronic. When the amount of chitosan was less than 0.001 parts by weight, the nanoparticles were not sufficiently coated with chitosan, making it difficult to exhibit positive charges on the surface. More than 200 parts by weight of chitosan caused the nanocapsules to be too large in size or to precipitate partially. In the subsequent experiments, the pluronic was mixed with chitosan in equal amounts.

As shown in FIG. 3, for particle size (a), when coated with chitosan having a molecular weight of 20 kDa, the nanoparticles were formed into chitosan-coated nanocapsules ChiNC (ChiNC 3K, ChiNC 10K, and ChiNC 20K) that were 100 nm or less in diameter while the application of chitosan having a molecular weight of 50 kDa and 100 kDa resulted in chitosan-coated nanocapsules ChiNC (ChiNC 50K and ChiNC 100K) which were about 200 nm in diameter. Turning to polydispersity (b), the chitosan-coated nanocapsules having a molecular weight of 20 kDa or less, ChiNC (ChiNC 3K, ChiNC 10K, and ChiNC 20K) were observed to have a polydispersity index of 0.2 or less while in the chitosan-coated nanocapsules having molecular weights of 50 kDa and 100 kDa, ChiNC (ChiNC 50K and ChiNC 100K), and a polydispersity index of about 0-2-0.3 was measured. For surface charge (c), PluNC itself, which is not coated with chitosan, was observed to take a negative charge of about -5 mV wherein, when coated with chitosan, the capsules were positively charged irrespective of the molecular weight thereof. In addition, morphological observation exhibited spherical structures in the nanocapsules, revealing that the nanocapsule increased in size with the increase of molecular weight in the chitosan (d).

Although not shown herein, chitosan with a molecular weight more than 100 kDa should be dissolved in acetic acid before being applied to the particles. However, when such a large chitosan was dissolved in acetic acid for coating, precipitation occurred, decreasing stability in the formulation.

From the result, it was understood that in the chitosan-coated nanocapsules of the present disclosure, chitosan can be stably coated on the nanoparticles composed of pluronics according to the preparation method and that the molecular weights of the chitosan to be applied have an influence on the size and dispersity of the nanocapsules. Furthermore, chitosan with a molecular weight of 3-20 kDa allowed the construction of nanocapsules that were small and homogenous in size and acquired formulation stability.

### Example 1-4. Optimization for preparation of chitosan-coated nanocapsule according to solvent

An optimization was established for the solvent used in the preparation method for chitosan-coated nanocapsules.

As selected solvents, acetone (ACE), ethanol (ETH), tetrahydrofuran (THF), chloroform (CHL), and dichloromethane (DCM) were assayed. Employing such solvents, platforms can be established to load various drugs (active agents) different in solubility in the solvents.

First, 20 mg of pluronic F127 was dissolved in 1 ml of each of acetone, ethanol, and tetrahydrofuran according to nanoprecipitation, followed by reaction for 2 hours. Then, the reaction mixture was slowly dropped into 4 ml of deionized water that was being stirred at 400 rpm to give polymeric nanocapsules from which the solvents were then removed for 6 hours under a hood. Finally, 20 mg of chitosan (deacetylation 90%, molecular weight 10 kDa) was added to the nanocapsules from each solvent and stirred at room temperature for 2 hours to afford chitosan-coated nanocapsules.

For chloroform, single emulsion was employed. A solution of 20 mg of pluronic F127 in 1 ml of chloroform was subjected to reaction for 2 hours. Afterward, the reaction mixture was slowly dropped to 4 ml of deionized water that was being stirred at 400 rpm to give nanoparticles which were then dispersed using a homogenizer before the solvent was removed for 2 hours through vacuum drying. Finally, 20 mg of chitosan (deacetylation 90%, molecular weight 10 kDa) was added to the polymeric nanocapsules and stirred at room temperature for 2 hours to afford chitosan-coated nanocapsules.

The 10 kDa chitosan-coated nanocapsules (ChiNC 10K) prepared according to nanoprecipitation and single emulsion were analyzed for size, dispersity, and surface charge, using an electrophoretic light scattering spectrophotometer (ELS-Z2, Otsuka).

As can be seen in FIG. 4, the chitosan nanocapsules (ChiNC 10K), prepared by nanoprecipitation using the organic solvents acetone (ACE), ethanol (ETH), and tetrahydrofuran (THF), which are all of high water miscibility, were all observed to be 60 nm in size and have a polydispersity index (PDI) of 0.3 or less, exhibiting monodispersity.

In contrast, the chitosan-coated nanocapsules (ChiNC 10K) prepared by single emulsion using chloroform (CHL), which is an organic solvent with poor water miscibility, were about 750 nm in size (same as in dichloromethane) and exhibited a large polydispersity index (PDI), compared to the chitosan-coated nanocapsules prepared by nanoprecipitation using acetone (ACE), ethanol (ETH), and tetrahydrofuran (THF). However, all of the chitosan-coated nanocapsule groups took a surface charge of about 20 mV, demonstrating that the polymeric capsules coated with chitosan, that is, chitosan-coated nanocapsules can be stably constructed using various solvents and processes.

### Example 1-5. Establishment of preparation method for drug-loaded nanocapsule

Chitosan-coated nanocapsules loading a drug therein were prepared by nanoprecipitation and membrane resuspension and characterized according to the preparation methods. In this regard, the drug was the insoluble substance cyclosporine (hereinafter referred to as "CsA").

In the membrane resuspension, 0.2 mg (2 wt%) or 0.6 mg (6 wt%) of CsA was dissolved, together with 10 mg of pluronic F126, in 1 ml of acetone and stirred at room temperature for 2 hours to give a reaction solution. The reaction solution was stood for 2 hours under a fume hood to form a membrane with the evaporation of acetone. The formed membrane was added with 5 ml of deionized water, stirred for 30 minutes or longer, and mixed with the same weight of 10 kDa chitosan as that of the pluronic to afford nanocapsule.

In another method nanoprecipitation, 0.2 mg or 0.6 mg of CsA was stirred, together with 10 mg of pluronic F126, in 1 ml of acetone at room temperature for 2 hours to give a reaction solution which was then slowly dropped to 4 ml of deionized water that was being stirred at 530 rpm. The mixture was stirred for 4 hours under a fume hood so that the acetone spontaneously vaporized. To the deionized water containing the acetone-removed nanoparticles composed of the pluronic, 10 kDa chitosan was added in the same amount as in the pluronic, followed by stirring to afford nanocapsules. The nanocapsules were measured for particle size, using a particle size analyzer and transmission electron microscopy. The results are depicted in FIG. 5.

As can be seen in FIG. 5, CsA-lacking nanocapsules were about 2-fold smaller in size when prepared by nanoprecipitation than by membrane resuspension. The CsA-containing nanocapsules, when prepared by membrane resuspension, were largely different in particle size from each other depending on the content of CsA. The nanocapsules partially aggregated at a CsA content of 6 wt%. In contrast, a difference in particle size according to CsA contents among the CsA-containing nanocapsules prepared by nanoprecipitation was not large, relative to that among nanoparticles prepared by membrane resuspension, whereby nanocapsules could be stably constructed within a certain size range, without aggregation. The capsules were measured to have a size of 100 nm or less at a CsA content of 2 wt% and a size of 200 nm or less at a CsA content of 6 wt%.

The data indicate that nanoprecipitation is preferably applied to the construction of the chitosan-coated nanocapsules having a drug loaded therein according to the present disclosure in order to improve the stability of the formulation.

### Example 1-6. Ratio of solvent and distilled water in nanoprecipitation

The nanocapsules prepared by nanoprecipitation were measured for size according to the ratio between the solvent acetone of the reaction solution and the deionized water used in preparing the nanoparticles during the procedure of Example 1-5.

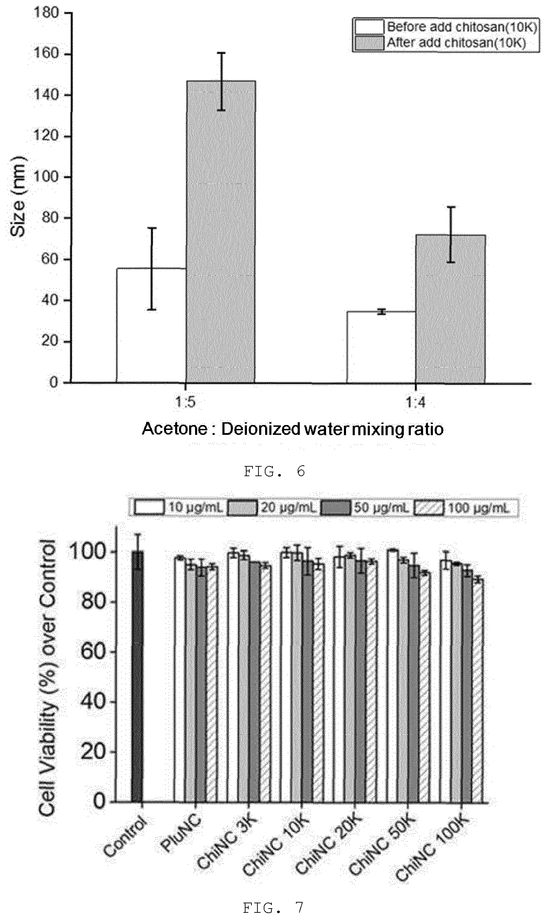

Chitosan-coated nanocapsules having a drug loaded therein were prepared in the same manner as the nanoprecipitation in Example 1-5, with the exception that the drug was loaded at a content of 6 wt%, with acetone and deionized water mixed at a ratio of 1:5 or 1:4. The nanocapsules were analyzed for particle size and the result is depicted in FIG. 6. In this regard, sizes of nanocapsules before and after coating with chitosan were measured.

As shown in FIG. 6, the nanocapsules were formed to have a size of 100 nm or less both before and after chitosan addition when acetone was mixed at a ratio of 1:4 with deionized water, as opposed to the nanocapsules formed at a ratio of 1:5 of acetone: deionized water.

The result implies that the chitosan-coated nanocapsules having a drug loaded therein according to the present disclosure can be prepared to have a size for stability by nanoprecipitation using a solvent and deionized water at a ratio of 1:4.

Although not shown herein, the solvents DMSO (dimethyl sulfoxide), ethanol, acetonitrile, tetrahydrofuran, chloroform, and dichloromethane could also allow the construction of nanocapsules in stable sizes when they were used at a ratio of 1:4 with deionized water (see Example 1-4).

### <EXAMPLE 2. Assay for Cytotoxicity of Chitosan-Coated Nanocapsule>

The chitosan-coated nanocapsules (ChiNC) prepared in Example 1-3 were assayed for cytotoxicity.

NIH3T3 cells were seeded at a density of 10,000 cells per well into 96-well plates and incubated for 8-12 hours. Then, the cells were treated with 10 pg/ml, 20 pg/ml, 50 µg/ml, or 100 pg/ml PluNC or ChiNC, both prepared in Example 1-3, for 24 hours and analyzed for cell viability using CCK8 (cell counting kit-8) according to the manufacturer's manual. The result is depicted in FIG. 7. In this regard, the cell viability for each treated group was expressed relative to 100% for the non-treated NIH3T3 cells as a control.

As shown in FIG. 7, PluNC or ChiNC allowed a cell viability of 90% or higher at all concentrations.

The data demonstrates that the chitosan-coated nanocapsules of the present disclosure are highly biocompatible without cytotoxicity.

### <EXAMPLE 3. Preparation Condition for Chitosan-Coated Nanocapsule Having Insoluble (Fat-Soluble) Active Agent Loaded therein>

In the present disclosure, preparation conditions were established for chitosan-coated nanocapsules containing active agents, especially, fat-soluble anticancer agents (paclitaxel, docetaxel), anti-inflammatory agents (dexamethasone), immunosuppressants (cyclosporin A), antioxidants (tocopheryl acetate), anti-wrinkle agents (retinyl palmitate), anti-hair loss preparations (minoxidil, finasteride), and anti-aging agents (tocopheryl acetate, retinyl palmitate).

A solution of docetaxel, paclitaxel, dexamethasone, tocopheryl acetate, cyclosporin A, or retinyl palmitate in 1 ml of acetone was mixed and reacted with 20 mg of pluronic F127 for 2 hours. Then, the reaction mixture was slowly dropped to 4 ml of deionized water that was being stirred at 400 rpm to prepare drug-loaded polymeric nanocapsules from which the acetone was then removed for 6 hours under a hood. Finally, 20 mg of chitosan (deacetylation 90%, molecular weight 10 kDa) was added to the polymeric nanocapsules and stirred for 2 hours at room temperature to afford chitosan-coated nanocapsules having the drug loaded therein. The drugs which remained unloaded were removed by ultrafiltration (Amicon Ultra-15 filter).

Separately, a solution of finasteride, minoxidil, tocopheryl acetate, or retinyl palmitate in 1 ml of ethanol was mixed and reacted with 20 mg of pluronic F127 for 2 hours. Then, the reaction mixture was slowly dropped to 4 ml of deionized water that was being stirred at 400 rpm to prepare drug-loaded polymeric nanocapsules from which the ethanol was then removed for 6 hours under a hood. Finally, 20 mg of chitosan (deacetylation 90%, molecular weight 10 kDa) was added to the polymeric nanocapsules and stirred for 2 hours at room temperature to afford chitosan-coated nanocapsules having the drug loaded therein. The drugs which remained unloaded were removed by ultrafiltration (Amicon Ultra-15 filter).

The drug-loaded ChiNC 10K thus prepared was analyzed for size, dispersity, and surface charge using an electrophoretic light scattering spectrophotometer (ELS-Z2, Otsuka).

### Example 3-1. Preparation of chitosan-coated nanocapsules having paclitaxel and docetaxel loaded therein

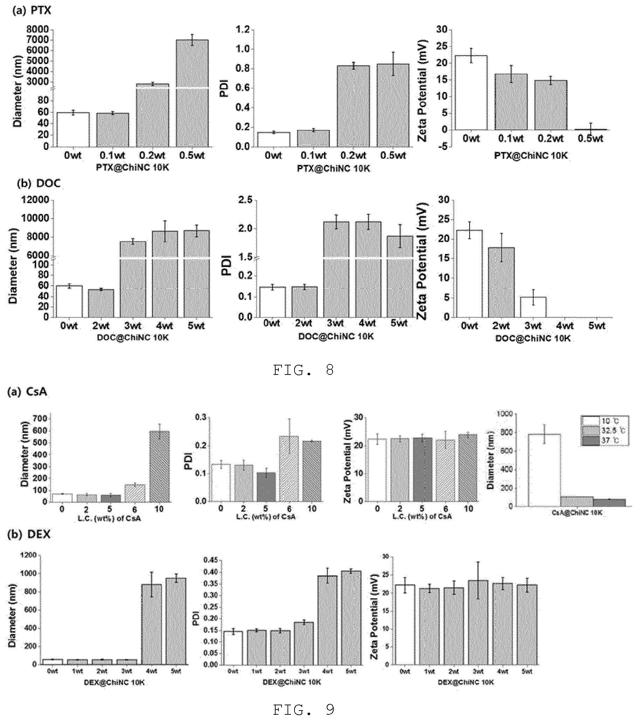

As shown in FIG. 8, paclitaxel (PTX)-loaded nanocapsules could be prepared and the load did not have significant influence on size, dispersity, and surface charge until reaching up to 0.1 wt%. At a load content of 0.2 wt%, the nanocapsules expanded to a diameter of up to 3 µm, but did not precipitate. Thus, it was found that chitosan-coated microcapsules having the drug loaded at the content therein can be prepared for topical application. However, partial precipitation occurred when the drug was loaded at a content of 0.5 wt%.

Docetaxel (DOC) was observed to be stably loaded at a content of up to 2 wt% into the chitosan-coated nanocapsules. In addition, formulation conditions that guarantee the loading of the drug at a content of up to 3 wt% without precipitation were figured out. Thus, chitosan-coated nanocapsules and chitosan-coated microcapsules, each having an anticancer agent loaded therein, could be prepared.

### Example 3-2. Preparation of chitosan-coated nanocapsule having cyclosporin A and dexamethasone loaded therein

As can be seen in FIG. 9, Cyclosporin A (CsA) was stably loaded at a content of up to 5 wt% in chitosan-coated nanocapsules. In addition, formulation conditions that guaranteed the loading of the drug at a content of up to 10 wt% without precipitation were figured out. At a load content of 5 wt%, chitosan-coated nanocapsules were obtained. In consideration of the applicability of slightly larger chitosan-coated nanocapsules to topical therapy, the drug could be loaded at a content of up to 10 wt%. In addition, an assay for temperature sensitivity revealed that the chitosan-coated nanocapsules had a particle size of 700 nm or more at 10°C and 100 nm or less (30-100 nm) at 32.5°C or 37°C.

Dexamethasone (DEX) could be loaded optimally at a content of up to 3 wt% and no precipitation took place at a load content of up to 5 wt%, so that chitosan-coated nanocapsules with a size of 900 nm could be prepared.

### Example 3-3. Preparation of chitosan-coated nanocapsule having retinyl palmitate and tocopheryl acetate loaded therein

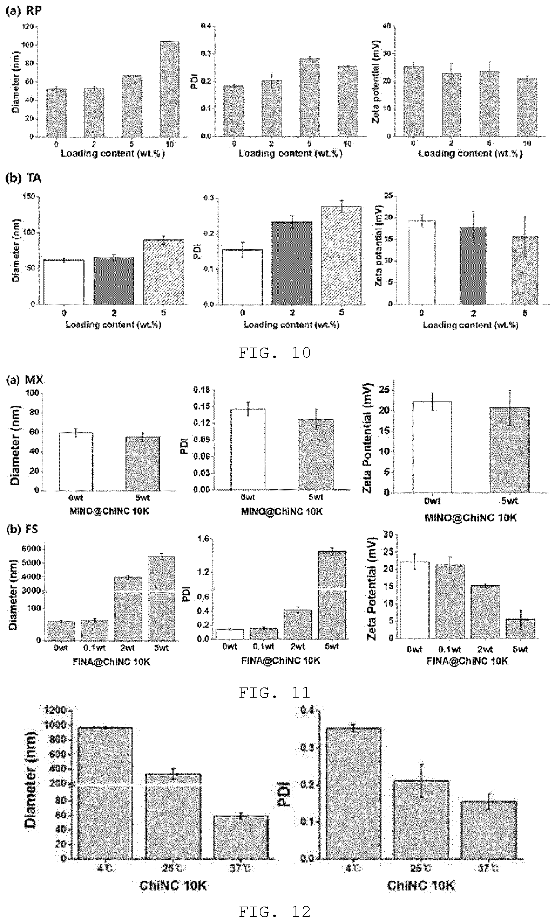

As shown in FIG. 10, retinyl palmitate (RP) was observed to be loaded stably at a content of up to 5 wt% in chitosan-coated nanocapsules. In addition, at a load content of 10 wt%, the chitosan-coated nanocapsules were as large as 100 nm in size, but remained unchanged in dispersity and surface charge. Thus, they were stably prepared at the load content, without precipitation.

As can be understood from data of Table 2, below, retinyl palmitate could be loaded as an active agent at a content of as high as 20 wt% because the nanocapsules, although increasing in size, were not largely different in terms of dispersity and surface charge at the loading content.

**TABLE 2**

| Loading Content (wt%) | size (nm) | PDI | Charge |
|---|---|---|---|
| 0 | 52 | 0.18 | 25.3 |
| 2 | 53 | 0.20 | 22.8 |
| 5 | 67 | 0.28 | 23.6 |
| 10 | 104 | 0.25 | 20.8 |
| 20 | 254 | 0.31 | 17.5 |

As for tocopheryl acetate (TA), its optimal loading content was up to 2 wt%, with no precipitation occurring at up to 5 wt%. Thus, chitosan-coated nanocapsules 90 nm in size could be prepared, with the drug loaded therein.

### Example 3-4. Preparation of chitosan-coated nanocapsule having minoxidil and finasteride loaded therein

As can be seen in FIG. 11, minoxidil (MX) did not significantly affect the size, dispersity, and surface charge of the chitosan-coated nanocapsules until its loading content reached 5 wt% and thus could be optionally loaded into the chitosan-coated nanocapsules.

In addition, finasteride (FS) could be optimally loaded at a content of up to 0.1 wt% and did not cause precipitation at a content of up to 2 wt%, thereby allowing the preparation of chitosan-coated microcapsules as large as 4 µm in size. In addition, the chitosan-coated nanocapsules having the drug loaded at a content of 5 wt% therein were greatly different in terms of dispersity and surface charge from the chitosan-coated nanocapsules having no drugs loaded therein. Thus, an optimal condition for the drug was up to 0.1 wt%. For use in topical delivery through microcapsules, the drug could be loaded at a content of as large as 2 wt%.

As stated in the foregoing, preparation conditions were established for chitosan-coated nanocapsules 100 nm or less in size to which insoluble (fat-soluble) active agents were loaded at a content ranging from 0.1 wt% to 20 wt%. Accordingly, they could be applied to optimization for preparation conditions of chitosan-coated nanocapsules to which active agents such as tetradrine, astaxanthin, curcumin, ascorbyl palmitate, caffeic acid phenethyl ester (CAPE), centella asiatica, beta-sitosterol, ascorbyl tetraisopalmitate, tripeptide collagen, and so on, were loaded.

### <EXAMPLE 4. Preparation Condition for Chitosan-Coated Nanocapsule Having Water-Soluble Active Agent Loaded therein>

In the present disclosure, preparation conditions were established for chitosan-coated nanocapsules to which water-soluble drugs including anticancer agents (doxorubicin), immunogens (ovalbumin), proteins as therapeutic agents, and medicines (bovine serum albumin, BSA) were loaded.

A solution of 20 mg of pluronic F127 in 1 ml of acetone was subjected to reaction for 2 hours. Then, the reaction solution was slowly dropped to 4 ml of deionized water that was being stirred at 400 rpm to give polymeric nanoparticles from which the acetone was removed for 6 hours under a hood. Finally, 20 mg of chitosan (deacetylation 90%, molecular weight 10 kDa) were added to the polymeric nanoparticles and stirred at room temperature for 2 hours to afford chitosan-coated nanocapsules.

A water-soluble drug (doxorubicin, BSA, or ovalbumin) was added to the chitosan-coated nanocapsules thus obtained, and then incubated at 4°C for 2 hours. The drugs that remained unloaded were removed by ultrafiltration (Amicon Ultra-15 filter), followed by optimization for loading conditions.

Unloaded drugs were analyzed by absorbance (480 nm) for doxorubicin and by absorbance (580 nm) through Coomassie blue assay for BSA and ovalbumin.

The chitosan-coated nanocapsules (ChiNC 10K) having the water-soluble drugs loaded thereto were analyzed for size, dispersity, and surface charge by an electrophoretic light scattering spectrophotometer (ELS-Z2, Otsuka).

### Example 4-1. Preparation of chitosan-coated nanocapsule having doxorubicin loaded thereto

Unlike fat-soluble drugs, water-soluble drugs were loaded at a low temperature (4°C) using temperature-responsive characteristics of the chitosan-coated nanocapsules (volume expansion to a size of 1 µm at 4°C) as shown in FIG. 12.

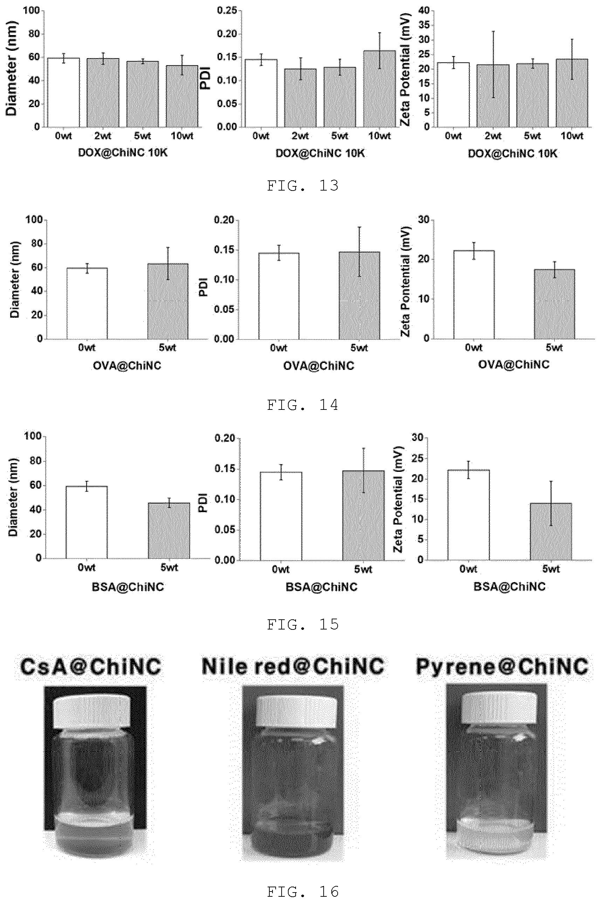

The chitosan-coated nanocapsules having doxorubicin (DOX) loaded thereto were similar to each other in terms of all of the size, dispersity, and surface charge, as shown in FIG. 13. Particularly, the drug could be stably loaded at a content of up to 10 wt%. Although not shown in FIG. 13, microcapsules could be formed even at a loading content of 20 wt%.

An optimal loading content was observed to be 6 wt% as measured by an absorbance assay.

### Example 4-2. Preparation of chitosan-coated nanocapsules having ovalbumin loaded therein

As shown in FIG. 14, chitosan-coated nanocapsules having ovalbumin (OVA) loaded thereto were similar to each other in terms of size, dispersity, and surface charge, without precipitation, until the loading content of 5 wt%. An absorbance assay revealed that a loading content of 3 wt% was optimal.

### Example 4-3. Preparation of chitosan-coated nanocapsule having bovine serum albumin (BSA) loaded thereto

As shown in FIG. 15, chitosan-coated nanocapsules were similar in terms of size, dispersity, and surface charge until BSA (67 kDa), which is a model drug for various proteins as therapeutic agents, was loaded at a content of up to 5 wt%. The nanocapsules allowed the drug to be stably loaded thereto, without precipitation. An optimal loading content was observed to be 4 wt% as measured by an absorbance assay.

As stated in the foregoing, preparation conditions were established for chitosan-coated nanocapsules of 100 nm or less in size to which water-soluble active agents were loaded at a content ranging from 0.1 wt% to 20 wt%. Accordingly, they could be applied to optimization for preparation conditions of chitosan-coated nanocapsules to which active agents such as phospholipase A2 (PLA2), basic fibroblast growth factor (b-FGF), vascular endothelial growth factor (VEGF) and so on were loaded.

### <EXAMPLE 5. Formulation Stability of Chitosan-Coated Nanocapsule Having Drug Loaded thereto>

### Example 5-1. Formulation stability of chitosan-coated nanocapsule having drug loaded therein

On the basis of the established preparation method and condition for chitosan-coated nanocapsules in Example 4, chitosan-coated nanocapsules having drugs loaded therein were prepared wherein insoluble cyclosporin A (CsA) was used as an insoluble drug and the commonly available drugs Nile red and pyrene were used as model insoluble drugs.

Together with 10 mg of pluronic F126, 0.2 mg or 0.5 mg of CsA, Nile red, or pyrene was added to 1 ml of acetone and mixed at room temperature for 2 hours to give a reaction mixture which was then slowly dropped to 4 ml of deionized water that was being stirred at 530 rpm. Stirring was continued for 4 hours under a fume hood to allow spontaneous vaporization of the acetone. After removal of acetone, the nanoparticles composed of the pluronic in deionized water were mixed with the same weight of 10 kDa chitosan as that of the pluronic to afford chitosan-coated nanocapsules having the drugs loaded therein (CsA@ChiNC, Nile red@ChiNC, and Pyrene@ChiNC, respectively). CsA@ChiNC, Nile red@ChiNC, and Pyrene@ChiNC were analyzed for stability by monitoring precipitation while they were stood at room temperature. The result is given in FIG. 16.

As can be seen in FIG. 16, neither of CsA@ChiNC, Nile red@ChiNC, and Pyrene@ChiNC precipitated.

From the data, it was understood that chitosan-coated nanocapsules having drugs loaded therein according to the present disclosure are stable in terms of formulation.

### Example 5-2. Lyophilization stability of chitosan-coated nanocapsule having drug loaded therein

Chitosan-coated nanocapsule formulations were assayed for stability, especially stability in deionized water and PBS and before and after lyophilization.

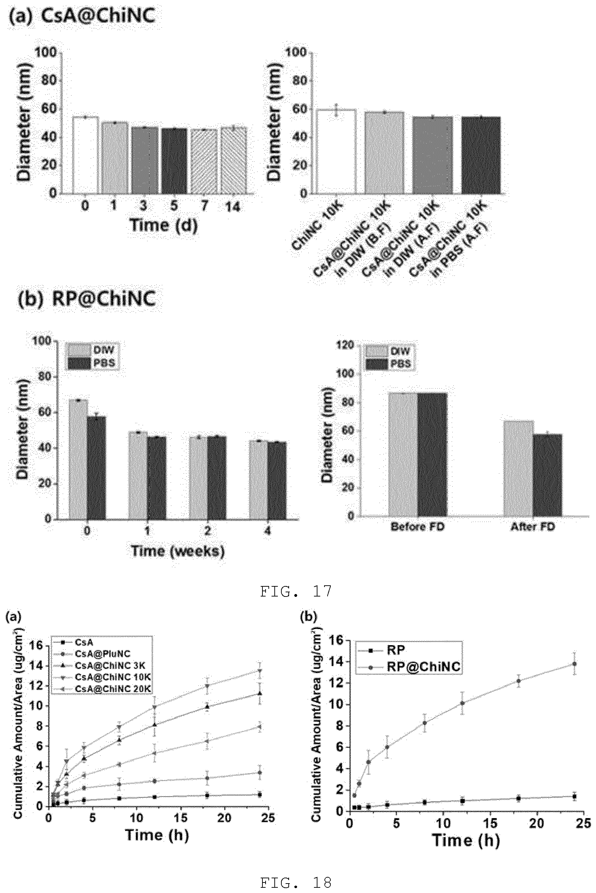

FIG. 17 showed stability of chitosan-coated nanocapsules having cyclosporin A and retinyl palmitate (RP) loaded therein as measured under the conditions of 37°C and 100 rpm. As can be seen, similar sizes were detected in both deionized water and PBS after 4 weeks, demonstrating that the nanocapsules can be stably maintained.

The high stability of the chitosan-coated nanocapsules was also confirmed as they were observed to undergo neither size changes nor precipitation before and after lyophilization (Before FD or B.F: before freeze-drying and After FD or A.F: after freeze-drying). Particularly, the nanocapsules were easy to resuspend even in the absence of a cryoprotectant during lyophilization.

### <EXAMPLE 6. Transdermal Drug Delivery Effect of Chitosan-Coated Nanocapsule Having Drug Loaded therein>

Transdermal delivery formulations for drugs or active ingredients have always been items attracting keen interest in the pharmaceutical and cosmetic fields. The chitosan-coated nanocapsules having drugs loaded therein according to the present disclosure were analyzed for drug delivery efficacy.

### Example 6-1. Optimization of Chitosan type for skin permeation by Franz diffusion cell system

In Example 1-3, the chitosan-coated nanoparticles with a molecular weight of 20 kDa or less (ChiNC 3K, ChiNC 10K, and ChiNC 20K) were discovered to stably form chitosan nanoparticles 100 nm or less in size. To determine which chitosan-coated nanoparticles are optimized for skin permeability, a Franz diffusion cell system was utilized.

In a Franz diffusion cell system, a formulation including a drug is applied to a donor chamber while a receptor chamber is filled with physiological saline such as PBS (phosphate buffered saline), with a permeable layer, such as a permeable membrane, animal skin or cell culture skin, fixed between the donor chamber and the receptor chamber. Skin permeability can be determined by measuring the amount of the drug diffusing from the donor chamber to the receptor chamber.

In a Franz diffusion cell system, 5 ml of PBS (pH 7.4 containing 0.05% polysorbate 80) was added to the receptor chamber, a human cadaver skin with a size of 1.5 x 1.5 cm was fixed between the receptor chamber and the donor chamber, and each sample was placed in a donor chamber. The conditions of 37°C and 600 rpm were set in the receptor chamber and 500 µl of the sample was recovered each time at sample times of 0.5, 1, 2, 4, 8, 12, 18, and 24 hours. The drug passing through the skin was quantitated according to time by HPLC. The result is depicted in FIG. 18.

As shown in FIG. 18, CsA exhibited the highest skin permeability when applied to chitosan-coated nanocapsules prepared from 10 kDa chitosan (ChiNC 10K). A statistically significant difference from 3kDa chitosan was evaluated to result from the higher surface charge of ChiNC 10K than ChiNC 3K as shown in FIG. 3.

Chitosan nanocapsules (ChiNC 10K) prepared from 10 kDa chitosan significantly increased in skin permeability compared to polymeric capsules without chitosan coating (PluNC), and were six or more times higher in skin permeability than the most commonly used liposome formulation.

As a result of evaluating the use of RP as a drug, RP exhibited 14 or more times higher skin permeability when loaded to the optimal condition ChiNC 10K than when RP itself was tested as a control, demonstrating that 10 kDa chitosan can improve skin permeability of drugs.

### Example 6-2. Measurement of skin permeability of chitosan-coated nanocapsule by using Franz diffusion cell system

Skin permeabilities of nanocapsules and chitosan-coated nanocapsules were measured using a Franz diffusion cell system in the same manner as in Example 6-1.

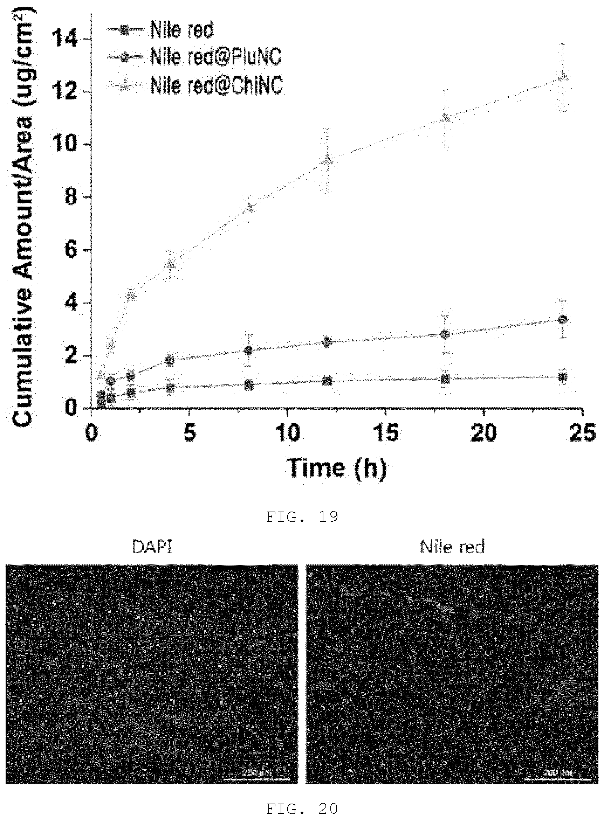

To a donor chamber, chitosan-coated nanocapsules containing 2 wt% of Nile red (Nile red@ChiNC) were added in an amount of 2 mg/ml. As a control, Nile red alone, or Nile red-containing nanocapsules without a chitosan coating (Nile red@PluNC) were employed. After the conditions of 37°C and 600 rpm were set for the receptor chamber, 500 µl of the sample was taken from the receptor chamber each time at sample times of 0.5, 1, 2, 3, 8, 12, and 24 hours. Fluorescence in the samples taken at the predetermined times was measured. The result is depicted in FIG. 19.

As shown in FIG. 19, Nile red alone almost did not penetrate into the skin whereas skin permeability was increased for use of Nile red@PluNC and Nile red@ChiNC. Particularly, Nile red@ChiNC four fold or more increased in skin permeability than Nile red@PluNC.

The results demonstrate excellent skin permeability of the chitosan-coated nanocapsules having drugs loaded therein according to the present disclosure.

### <EXAMPLE 7. Transdermal Delivery in Animal Model>

### Example 7-1. Transdermal delivery of Nile red through chitosan-coated nanocapsule in animal model

The chitosan-coated nanocapsules containing 2 wt% of Nile red (Nile red@ChiNC) prepared in Example 4 were controlled to have a concentration of 2 mg/ml and applied at a dose of 300 µl for 5 days to the shaved dorsal site of each mouse. Thereafter, the dorsal skin was sampled and fixed for 12 hours in 10% neutral formalin. The fixed skin was embedded in an OCT (optimal cutting temperature) compound and snap-frozen with liquid nitrogen at -20°C or lower, followed by cutting into 20 pm-thick blocks with a cryosection machine. The blocks were attached to slide glass to obtain skin tissue samples. They were washed with deionized water and a distribution of Nile red in the skin was observed under a fluorescence microscope. The result is depicted in FIG. 20.

As shown in FIG. 20, when Nile red@ChiNC was applied to the skin, Nile red was found within the skin tissue.

From the data, it could be understood that the chitosan-coated nanocapsules having drugs loaded therein according to the present disclosure can deliver the drugs through the skin.

### Example 7-2. Efficacy of drug through transdermal delivery

Cyclosporin A is used as a therapeutic agent for psoriasis and atopy and has been reported to have an effect on hair regrowth. However, cyclosporin A is limitedly delivered into the skin because it is insoluble to water due to the high hydrophobicity thereof. Thus, cyclosporin A should be dissolved in an organic solvent such as acetone before application to the skin. When applied to the skin, however, such an organic solvent may irritate the skin and cause a loss of the skin barrier.

Thus, effective penetration of cyclosporin A into the skin without skin irritation or injury can increase the hair regrowth efficacy thereof, resulting an excellent therapeutic effect on hair loss. Thus, the chitosan-coated nanocapsules according to the present disclosure were examined for transdermal delivery of cyclosporin A in terms of the hair regrowth effect thereof.

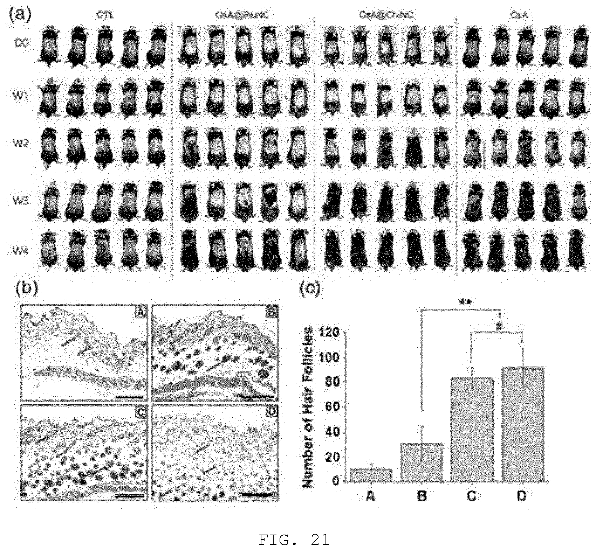

Hair regrowth experiments were conducted. In this regard, black C57B/6 female mice at 7 weeks of age had the back shaven according to the guideline of the platform Ministry of Food and Drug Safety. The shaved mice were divided into groups treated with: saline (control (CTL)); cyclosporin A in acetone (CsA); and chitosan-coated nanocapsules containing 5 wt% of cyclosporin A prepared in Example 4 (CsA@ChiNC). Each sample was applied five times a week for 4 weeks (at a single dose of 50 mg/kg for CsA). Hair regrowth effects were examined with the naked eye, and the results are depicted in FIG. 21.

As shown in FIG. 21, when treated with cyclosporine A in acetone (CsA), five mice were all observed to have hairs grown on week 4 at which a maximal hair regrowth effect could be obtained, whereas hairs were naturally grown at a rate of less than 5 % in the control (CTL). For the group treated with CsA itself, it was considered that the skin tissue and barrier were damaged by the acetone in which CsA is dissolved so that the CsA drug penetrated into the damaged skin.

Hair regrowth was observed in four among five mice in the CsA@ChiNC group. CsA@ChiNC exhibited a hair regrowth effect as high as 80% of that of CsA itself.

Additionally, dorsal skin samples were taken from the mice in each group and analyzed for numbers and sizes of follicles, and the results are depicted in FIGS. 21 (b) and (c).

As shown in FIGS. 21 (b) and (c), similar numbers and sizes of hair follicles were detected between groups treated with CsA in acetone (D) and CsA@ChiNC (C).

The data indicate that the chitosan-coated nanocapsules having drugs loaded therein according to the present disclosure are superb in terms of skin permeability and transdermal drug delivery efficiency and can be used as a platform technology for transferring insoluble drugs into the human and animal bodies.

### <EXAMPLE 8. Increase in Bioavailability of Active Ingredient by Oral Administration of Nanocapsule>

Nanocapsules having cyclosporin A (CsA) loaded therein were orally administered to male Sprague-Dawley rats (200-250 g) . In this regard, nanocapsules having CsA loaded at 5 wt% therein (1 ml deionized water, 50 mg/kg) were administered using a feeding needle. Blood was sampled 2, 4, 6, 8, 12, and 24 hours after administration, and quantitatively analyzed by HPLC.

The blood half-life and bioavailability of CsA was increased in the group treated with the chitosan-coated nanocapsules, compared to the group treated with nanocapsules lacking chitosan.

In addition, for use in a hair regrowth experiment, black C57B/6 female mice at 7 weeks of age had the dorsal site shaven according to the guideline of the Ministry of Food and Drug Safety in the same manner as in Example 7. CsA and CsA@ChiNC solutions were orally administered three time a week for 4 weeks as described above. Higher hair regrowth effects were observed in the CsA@ChiNC-administered group than in the CsA-administered group. This result was considered to be attributed to the bioavailability improved by the chitosan-coated nanocapsules, demonstrating that the drug delivery system utilizing the chitosan-coated nanocapsules of the present disclosure has an excellent drug delivery effect.

## Claims

1. A nanocapsule, in which a nanoparticle comprising an active agent and a pluronic is coated with chitosan.

2. The nanocapsule of claim 1, wherein the nanocapsule is manufactured by a process comprising: a first step of preparing a reaction solution by dissolving an active agent and a pluronic in an organic solvent and reacting the same at room temperature;
a second step of preparing a nanoparticle by dropwise adding the reaction solution of the first step to distilled water, continuously stirring same, and removing the organic solvent of the reaction solution through natural evaporation; and
a third step of coating the nanoparticle of the second step by adding chitosan thereto.

3. The nanocapsule of claim 1 or 2, wherein the active agent is used in an amount of more than 0 part by weight to 20 parts by weight, based on 100 parts by weight of the pluronic.

4. The nanocapsule of claim 1 or 2, wherein the active agent is at least one selected from the group consisting of an anticancer agent, an immunosuppressant, an antioxidant, an anti-inflammatory agent, an anti-wrinkling agent, an anti-hair loss preparation, a wound healing agent, a skin whitening agent, a nutritional supplement, an immunogen, a protein as a therapeutic agent, a revascularization agent, an antifungal agent, an antibiotic, an antiviral agent, a sedative, an analgesic, an anti-aging agent, an anti-wrinkling agent, a skin whitening agent, a skin depigmenting agent, an ultraviolet blocking agent, a dye, a colorant, a deodorizing agent, and an air freshener.

5. The nanocapsule of claim 1 or 2, wherein the active agent is a fat-soluble or insoluble drug.

6. The nanocapsule of claim 5, wherein the fat-soluble or insoluble drug is at least one selected from the group consisting of paclitaxel, docetaxel, tetradrine, cyclosporin A, dexamethasone, tocopheryl acetate, astaxanthin, curcumin, ascorbyl palmitate, caffeic acid phenethyl ester (CAPE), retinyl palmitate, minoxidil, finasteride, centella asiatica, beta-sitosterol, ascorbyl tetraisopalmitate, and tripeptide collagen.

7. The nanocapsule of claim 1 or 2, wherein the active agent is a water-soluble drug.

8. The nanocapsule of claim 7, wherein the water-soluble drug is at least one selected from the group consisting of doxorubicin, phospholipase A2 (PLA2), ovalbumin, bovine serum albumin, basic fibroblast growth factor (b-FGF), and vascular endothelial growth factor (VEGF).

9. The nanocapsule of claim 1 or 2, wherein the pluronic is at least one selected from the group consisting of pluronic L35, pluronic L43, pluronic L44, pluronic L64, pluronic F68, pluronic P84, pluronic P85, pluronic F87, pluronic F88, pluronic F98, pluronic P103, pluronic P104, pluronic P105, pluronic F108, pluronic P123, and pluronic F127.

10. The nanocapsule of claim 1 or 2, wherein the nanocapsule has a particle size of 5-80 nm at 32.5-37°C.

11. The nanocapsule of claim 1 or 2, wherein the chitosan has a molecular weight of 3-100 kDa.

12. The nanocapsule of claim 1 or 2, wherein the chitosan is used in an amount of 0.001-200 parts by weight, based on 100 parts by weight of the pluronic.

13. The nanocapsule of claim 1 or 2, wherein the nanocapsule has a particle size of 30-500 nm at 32.5-37°C.

14. The nanocapsule of claim 1 or 2, wherein the nanocapsule has a particle size of 30-300 nm at 32.5-37°C.

15. The nanocapsule of claim 1 or 2, wherein the nanocapsule has a particle size of 30-100 nm at 32.5-37°C.

16. The nanocapsule of claim 2, wherein the organic solvent in the first step is at least one selected from the group consisting of acetone, DMSO (dimethyl sulfoxide), ethanol, acetonitrile, tetrahydrofuran, chloroform, and dichloromethane.

17. The nanocapsule of claim 2, wherein the distilled water in the second step has a volume 4 fold larger than that of the organic solvent in the first step.

18. The nanocapsule of claim 1 or 2, wherein the nanocapsule increases in skin permeability by two fold or more, compared to the active agent alone.

19. A drug delivery system, comprising the nanocapsule of claim 1 or 2.

20. The drug delivery system of claim 19, wherein the drug delivery system is an external skin preparation or an oral formulation.

21. A cosmetic composition, comprising the nanocapsule of claim 1 or 2.

22. A health function food composition, comprising the nanocapsule of claim 1 or 2.

23. A composition for medical devices, comprising the nanocapsule of claim 1 or 2.

24. A composition for daily necessities, comprising the nanocapsule of claim 1 or 2.
